# EUROPEAN PATENT APPLICATION

(11) **EP 4 015 509 A1**
(43) Date of publication of application: **22.06.2022**
(21) Application number: 20383124.3
(22) Date of filing: 18.12.2020
(51) Int. Cl.: C07D 257/02, A61K 49/00, A61K 51/00, C07F 5/00

(54) **ORGANOMETALLIC COMPOUNDS AND THEIR USE AS MULTIMODAL CONTRASTING MEDIA FOR IMAGING DIAGNOSIS**

(71) Applicant: Justesa Imagen S.A.U, 28823 Coslada (ES)
(72) Inventor: ALONSO SILVA, Ignacio, 28823 Madrid (ES); MARTÍN MARTÍN, Felix Ramón, 28823 Madrid (ES); CHICHARRO MARTÍN, Roberto, 28823 Madrid (ES); GARCÍA RAMOS, Álvaro, 28823 Madrid (ES); GARCÍA SALADO, Irene, 28823 Madrid (ES)
(74) Representative: Elzaburu S.L.P.

(57) **Abstract**

The present invention concerns a family of organometallic compounds of formula (I): 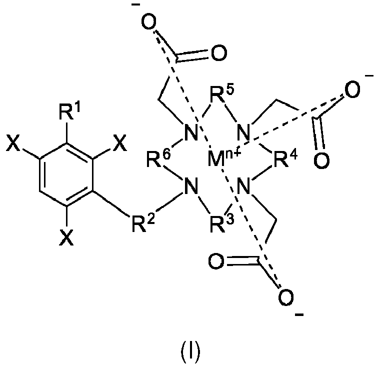 and its use to obtain contrast media applicable in various diagnostic imaging techniques such as X-ray, MRI, PET or SPECT or combinations of these techniques.

## Description

### FIELD OF THE INVENTION

The present invention refers to a family of compounds which can be used as contrast media in a combination of diagnostic imaging techniques or in any of these separately. This family of contrast media may have an accumulation in the liver and gallbladder, allowing their visualization in cholangiography and cholecystography studies. Additionally, another function of these contrast media is to act as a contrast agent for the intravascular space (*Blood Pool Agents*) in angiography studies. Therefore, the invention is included in the field of pharmaceutical chemistry and pharmacology.

### BACKGROUND OF THE INVENTION

The different diagnostic techniques of X-ray imaging, magnetic resonance imaging, positron emission tomography (*Photon Emission Tomography*: PET) or *single photon* emission *computed tomography* (SPECT) are widely used and there have been significant advances in this field in recent decades.

Suitable contrast media are required for each of these techniques. lodinated contrast media are widely used in X-ray. The most commonly used iodinated contrast media are triiodobenzoic acid derivatives, which in their ionic form as sodium or meglumine salt, are highly water-soluble compounds with a high osmolality. However, they have as disadvantages to cause adverse effects such as pain or burning sensation after intravenous administration due to their great difference in osmolality compared to blood. To solve these problems, non-ionic contrast media began to be used, with metrizamide being the first one approved. Among these compounds, iohexol, a derivative of aminoisophtalic acid, is the most used. lohexol, due to its great solubility in water, a high concentration of iodine and a lower osmolality, minimizes the adverse effects of this type of contrast media. Other contrast agents are iopanoic acid or its butyrate derivative, iocetamic acid or meglumine iotroxate dimer. These products have disadvantages such as an incomplete absorption, certain levels of toxicity, etc. The documents WO1993010078A1, WO1994022811A1 and WO1995026331A1 describe the use of certain iodinated dimers in X-ray diagnostic techniques.

In the field of magnetic resonance imaging (MRI), gadopentetate meglumine, a gadolinium-based compound, gadoxetic acid, gadobenate meglumine and gadofosveset are used as contrast media. There is a problem with certain contrast media that have a linear chelate structure, which can lead to an accumulation of free gadolinium in the brain. As an alternative, macrocyclic chelates are used, which are structurally very rigid, making it difficult to release the gadolinium cation. These include gadobutrol, meglumine gadoterate and gadoteridol. In nuclear medicine techniques such as PET, [¹⁸F]-fluorodeoxyglucose (FDG) is usually used.

In the last decades, the field of diagnostic imaging has undergone several developments related to the increased applicability of the conventional techniques above discussed. This, due to an increasingly solid knowledge of these techniques, added to technological advances such as the creation of new equipment, has risen to the possibility of using several combined techniques, improving the quality of the studied image. In this way, limitations that the techniques have when used independently are solved, such as the lack of anatomical information when PET or SPECT are used, or the very high dose that needs to be used in X-ray, among others.

In addition to the improvement of the techniques used in the field of diagnostic imaging, another object of study of the development in this field that is in continuous evolution is the manufacture of new contrast media to solve the problems presented by the already existing contrast media, and to improve certain characteristics such as the signal/noise ratio or the reduction of adverse effects that may occur after their administration. However, there are limitations related to the absence of contrast media designed to be used for the various combinations of possible techniques.

### DESCRIPTION OF THE INVENTION

In the present invention, using the information from these techniques, in addition to their limitations, new compounds are proposed to be used in a single technique or in a combination of several techniques. The chemical structure of the family of compounds to be claimed is formed by a chelate of a divalent or trivalent metal. The chelate is bonded by a linker to a benzene ring, which has several atoms of iodine and the 5 position is not substituted, leading to a great capacity of binding to macromolecules present in the bloodstream such as albumin.

In a first aspect, the present invention relates to a compound of formula (I): 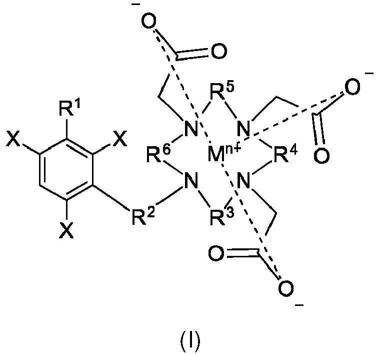 where
R¹ is selected from alkyl C₁-C₁₂, alkenyl C₂-C₁₂, aryl C₅-C₁₀, heteroaryl C₅-C₁₀, halogen, -OR⁷, -NR⁷R⁸, -CN, -C(O)R⁷, -C(O)OR⁷, -OC(O)R⁷, -C(O)NR⁷R⁸, -NHC(O)R⁷, -SO₂R⁷, - SO₂NR⁷R⁸ or -NO₂, where R⁷ and R⁸ are selected independently from H, alkyl C₁-C₁₂, alkenyl C₂-C₁₂, aryl C₅-C₁₀ or heteroaryl C₅-C₁₀;
X is a halogen, preferably bromine or iodine;
R² is the following group -(CH₂)ₘW(CH₂)ₚY(CH₂)_{q}Z(CH₂)ᵣ-, where each element (CH₂), W, Y and Z can be connected by a single or multiple bond to the adjacent element(s) depending on the group that are W, Y and Z, where m, p, q and r are integers independently selected from 0 to 10, and W, Y, Z are selected independently from the following groups: -O-, -NR⁹-, -C(O)-, -C(O)O-, -C(O)NR¹⁰-, -NR¹¹C(O)-, -CH₂-, -CH=, - SO₂-, -SO₂N(R¹²)- , aryl C₅-C₁₀ or heteroaryl C₅-C₁₀, with R⁹, R¹⁰, R¹¹ and R¹² independently selected from H or alkyl C₁-C₆;
R³, R⁴, R⁵ and R⁶ are independently selected from alkyl C₁-C₁₂, alkenyl C₂-C₁₂, aryl C₅-C₁₀ or heteroaryl C₅-C₁₀;
M is a metal that is selected from gadolinium, gallium, europium, ytterbium, dysprosium, manganese, strontium, thallium, lutetium, tungsten, germanium, copper, yttrium, indium, scandium or any of their possible isotopes, and n can be 2 or 3;
and its pharmaceutically acceptable salts, isomers and solvates.

The term "alkyl" refers, in the present invention, to hydrocarbon chain radicals, linear or branched, having between 1 and 12 carbon atoms, bonded to the rest of the molecule by a single bond, for example methyl, ethyl, *n*-propyl, *i*-propyl, *n*-butyl, *tert*-butyl, *sec-*butyl, *n*-pentyl, etc. These alkyl groups can be replaced by one or more substituents such as halogens, hydroxyl, alkoxyl, carboxyl, carbonyl, cyan, acyl, alkoxycarbonyl, amino, nitro, mercapto and alkylthio

The term "alkenyl" refers, in the present invention, to hydrocarbon chain radicals, linear or branched, having between 2 and 12 carbon atoms, and containing one or more double carbon-carbon bonds, e.g. vinyl, 1-propenyl, allyl, isoprenyl, 2-butenyl, 1,3-butadienyl etc. The alkenyl radicals can optionally be replaced by one or more groups such as halogens, hydroxyl, alkoxyl, carboxyl, carbonyl, cyan, acyl, alkoxycarbonyl, amino, nitro, mercapto and alkylthio.

The term "aryl" refers, in the present invention, to single or multiple aromatic rings, which have between 5 and 10 bonds in which a proton has been removed from the ring. Preferably the aryl group has 5 to 7 carbon atoms. Aryl groups are, for example, but not limited to phenyl, naphthyl, diphenyl, indenyl, phenanthrene or anthracene. The aryl radicals can optionally be substituted by one or more substituents such as halogens, hydroxyl, alkoxyl, carboxyl, carbonyl, cyan, acyl, alkoxycarbonyl, amino, nitro, mercapto and alkylthio.

The term "heteroaryl" refers, in the present invention, to aryl moiety where at least one C atom has been replaced by an S, O or N heteroatom. Heteroaryl groups are, for example, but not limited to pyridyl, furanyl, imidazolyl, benzimidazolyl, pyrimidinyl, thienyl, quinolinyl, indolyl, thiazolyl. The aryl radicals can optionally be substituted by one or more substituents such as halogens, hydroxyl, alkoxyl, carboxyl, carbonyl, cyan, acyl, alkoxycarbonyl, amino, nitro, mercapto and alkylthio.

The term "halogen" refers to bromine (Br) or iodine (I) atoms, preferably iodine.

The compounds of the present invention represented by formula (I), and more specifically, the detailed compounds belonging to this general formula described above may include isomers, depending on the presence of multiple bonds (e.g. *Z, E*), including optical isomers or enantiomers, depending on the presence of chiral centres. Individual isomers, enantiomers or diastereoisomers and mixtures thereof are within the scope of the present invention. Individual enantiomers or diastereoisomers and mixtures thereof can be separated by conventional techniques.

Within the compounds of the present invention represented by the general formula (I), are included the compounds that differ only by the presence of one or more isotopically enriched atoms. Such is the case of the substitution of a hydrogen atom by deuterium or tritium, as well as the substitution of a carbon enriched in ¹³C or ¹⁴C or the introduction of a radioactive metal. More specifically, the compounds of the invention include the isotopes of the possible metals: ⁶⁷Ga, ⁶⁸Ga, ⁶⁹Ga, ⁷¹Ga, ¹⁵²Gd, ¹⁵⁴Gd, ¹⁵⁵Gd, ¹⁵⁶Gd, ¹⁵⁷Gd, ¹⁵⁸Gd and ¹⁶⁰Gd, ¹⁵¹Eu, ¹⁵³Eu, ¹⁵⁵Eu, ⁸⁸Y, ⁸⁹Y, ⁹¹Y, ¹⁵⁶Dy, ¹⁵⁸Dy, ¹⁶⁰Dy, ¹⁶¹Dy, ¹⁶²Dy, ¹⁶³Dy, ¹⁶⁴Dy, ⁵²Mn, ⁵³Mn, ⁵⁴Mn, ⁵⁵Mn, ⁸⁴Sr, ⁸⁶Sr, ⁸⁷Sr, ⁸⁸Sr, ²⁰³Tl, ²⁰⁴Tl, ²⁰⁵Tl, ¹⁸²W, ¹⁸³W, ¹⁸⁴W, ¹⁸⁶W, ⁷⁰Ge, ⁷²Ge, ⁷³Ge, ⁷⁴Ge, ⁷⁶Ge, ⁶³Cu, ⁶⁵Cu, ⁶⁷Cu, ¹⁶⁸Yb, ¹⁷⁰Yb, ¹⁷¹Yb, ¹⁷²Yb, ¹⁷³Yb, ¹⁷⁴Yb and ¹⁷⁶Yb, ¹¹¹In, ¹¹⁵In ¹⁷⁷Lu, ⁴⁶Sc, ⁴⁷Sc, ⁴⁸Sc and ⁴⁴Sc. The preferred isotopes of this invention are ⁶⁷Ga and ⁶⁸Ga.

The term "pharmaceutically acceptable salts or solvates" refers to the salts or solvates of these compounds, which when administered to a patient provide a similar response to that produced by the compounds described in this patent. Preferably, "pharmaceutically acceptable" refers to molecular entities and compositions that are physiologically tolerable and do not typically produce an allergic reaction or other adverse effects, such as gastric disorder, dizziness or similar, once administered to a patient. More preferably, the term "pharmaceutically acceptable" means that it can be approved by a regulatory agency of a federal or state government or listed in the European Pharmacopoeia or other generally recognised pharmacopoeia for the use in animals, and more particularly in humans.

The compounds used in the invention may be in crystalline or amorphous form, either as free compounds, or as solvates (hydrates, for example) and it is understood that both forms are within the scope of the present invention.

In a preferred embodiment, R¹ is selected from -NHC(O)R⁷ or -C(O)OR⁷, and R⁷ is a hydrogen or C₁-C₆ alkyl.

In another favourite embodiment, X is iodine.

In another favourite embodiment, m is 0.

In a preferred embodiment, W is selected from -C(O)NR¹⁰- or -NR¹¹C(O)-, being R¹⁰ and R¹¹ are preferably H.

In a preferred embodiment, Y is selected from -NR⁹- or -NR¹¹C(O)-, being R⁹ and R¹¹ preferably H.

In a preferred embodiment, Z is selected from -CH₂- or -NR¹¹C(O)-, being R¹¹ preferably H.

In another preferred embodiment, p is selected from 1 to 2 and/or q is selected from 0 to 2 and/or r is selected from 0 to 1.

Each of the integers representing m, p, q and r are chosen independently from the values 0, 1, 2, 3, 4, 5, 6, 7, 8, 9 and 10, and the possible combinations of these values are included.

In a preferred embodiment, R³, R⁴, R⁵ and R⁶ can be the same or different C₁-C₆ alkyl. In a more preferred embodiment, R³, R⁴, R⁵ and R⁶ are C2 alkyl.

In a preferred embodiment, the compound of formula (I) is selected from the following list:
- Gadolinium {10-{2-[2-(3-acetylamino-2,4,6-triiodophenylamino)aminoethyl]-2-oxoethyl}-1,4,7,10-tetraaza-1-cyclododecyl}-1,4,7-triacetate
- ⁶⁷Gallium {10-{2-[2-(3-acetylamino-2,4,6-triiodophenylamino)aminoethyl]-2-oxoethyl}-1,4,7,10-tetraaza-1-cyclododecyl}-1,4,7-triacetate
- ⁶⁸Gallium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Europium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Dysprosium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Ytterbium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Gadolinium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- ⁶⁷Gallium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- ⁶⁷Gallium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Europium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Dysprosium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Ytterbium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate

Another aspect of the invention relates to a pharmaceutical composition comprising a compound of formula (I) as described above together with at least one excipient, adjuvant and/or vehicle which are pharmaceutically acceptable.

The pharmaceutically acceptable excipients, adjuvants and vehicles that can be used in such a composition are those known to the experts in the field and commonly used in the preparation of pharmaceutical compositions.

The pharmaceutical composition in which the compounds of the general formula (I) are found, can be administered by any appropriate route of administration, for example: oral, rectal, intravenous or any other appropriate route of administration. Preferably, this route is the intravenous route, either by direct administration or by infusion. In the pharmaceutical composition of the invention, the compounds of formula (I) can be dissolved or suspended in water or a physiological solution at different concentrations depending on the technique individually used or the combination of techniques used from 0.0001 to 5 mol/L, being even more preferred a dissolution or suspension at a concentration between 0.05 and 0.50 mol/L. In particular, the doses are 0.1 mmol/Kg when using the MRI technique, 1.0 mmol/Kg when using the X-ray technique, and 40 nmol/Kg when using a nuclear medicine technique. In general, the concentrations of the compounds of formula (I) in the pharmaceutical compositions are those that can achieve an effective radioactive labelling that provides sufficient activity to obtain acceptable images for diagnosis.

Another aspect of the invention concerns to the compound of formula (I) as described above for the use as a drug.

Another aspect of the invention concerns to the compound of formula (I) as described above for the use in the treatment and/or diagnosis of tumours.

The compounds of general formula (I) of the invention act as carriers of radioactive isotopes in theragnostic, which is understood as a combination of diagnosis and therapy. The gamma or beta emissions of the compounds of the invention can act simultaneously to diagnose and treat certain types of tumours.

Another aspect of the invention refers to a contrast media for obtaining images of organs and/or tissues of the human or animal body using nuclear magnetic resonance, X-ray, PET or SPECT, or combinations of these techniques, comprising at least one compound of formula (I) as above described.

This contrast media used in the field of diagnostic imaging allows organs and other parts of the body to be visualised, such as the liver or bile ducts and the intravascular space, performing as a Blood Pool Agent.

The family of compounds with a general formula (I), having three atoms of a high molecular weight halogen, especially iodine, has a great capacity to absorb X-rays. In addition, the presence of a metal of high molecular weight such as gallium, gadolinium, europium or ytterbium, which also have the capacity to absorb X-rays, increases the signal obtained in X-ray. This effect provides a great advantage, due to the need for a lower dose for the exploration of the area of interest in a patient. Additionally, the presence of metals such as gadolinium or europium, because of their paramagnetic nature, are optimal for use in MRI. Gallium, in addition to its X-ray absorption, can be present in different states, either as natural gallium, gallium-68, which allows its use in PET, or as gallium-67, which allows its use in SPECT.

Compounds of general formula (I), having the 5 position in the benzene ring free, may have a high affinity for macromolecules found in the blood stream, such as albumin. In this way, when it binds to albumin, the time that the compound remains in the intravascular space increases, visualising this area of the human body with a great signal in time, performing as a Blood Pool Agent. As albumin is metabolised in the liver, the binding of formula (I) compounds to this protein, accumulate in the liver, allowing its visualisation. The compounds of general formula (I) are partially eliminated by the urinary system and are also excreted through the bile ducts, allowing their visualisation.

Another aspect of the invention concerns to a procedure for the preparation of compounds of formula (I) as previously defined, which comprises making a compound of general formula (II): 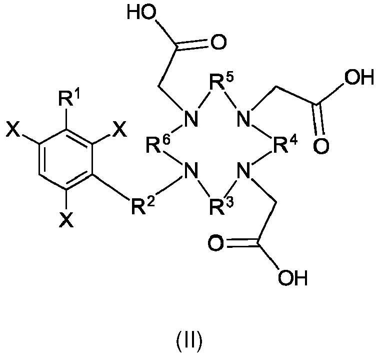 where R¹, R², R³, R⁴, R⁵ and R⁶ and X are defined as above,
to react with a metal that can be divalent or trivalent, and is selected from gadolinium, gallium, europium, ytterbium, dysprosium, manganese, strontium, thallium, lutetium, tungsten, germanium, copper, yttrium, indium, scandium or any of their possible isotopes, in the form of oxide or salt,
in the presence of a solvent, heating between 60 and 110 ºC, controlling the pH between 1.0 and 10.0 using a strong acid such as hydrochloric acid and a strong base such as sodium hydroxide

In a preferred embodiment, the solvent used is water or methanol, more preferably water.

In a more preferred embodiment, the temperature is set between 80 and 90 ºC.

In a more preferred embodiment, gadolinium oxide, gallium chloride, europium oxide, ytterbium oxide, dysprosium oxide, manganese chloride, etc. are used.

In a more preferred embodiment, radioactive elements such as gallium-67 and gallium-68 are introduced.

In a more preferred embodiment, the purification of the obtained product is carried out using ion exchange resin techniques, nanofiltration, electrodialysis or crystallization in organic solvents.

The compound of general formula (II) can be obtained by reacting a compound of general formula (III) with a compound of general formula (IV), where the compound of general formula (III) corresponds to: 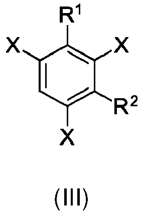 and R¹, R² and X are defined as above.

In a preferred embodiment, R² may contain a terminal leaving group which is a halogen, ester, acid chloride, mesylate, tosylate, triflate, among others.

And the compound of general formula (IV) corresponds to: 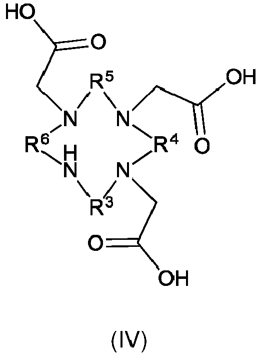 where R³, R⁴, R⁵ and R⁶ are defined as above.

The compounds of general formula (III) and (IV) react in the presence of a solvent, heating between 30 and 110 ºC, preferably 40 and 70 ºC.

In a preferred embodiment, when solvent is used, this is *N,N*-dimethylacetamide, tetrahydrofuran, ethyl acetate, water, methanol or a mixture of several solvents. In a more preferred embodiment, the mixture used is *N,N*-dimethylacetamide/water.

In a more preferred embodiment, the reaction is carried out in the presence of a base such as triethylamine.

In a preferred embodiment, the purification of the obtained product is carried out using ion exchange resin techniques, nanofiltration, electrodialysis or crystallization in organic solvents.

The compound with general formula (III) can be obtained by reacting a compound with general formula (V) with a linker defined as R², where the general compound (V) corresponds to:

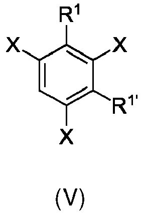

R¹ and X are defined as above and R^{1'} is defined as R¹.

In a preferred embodiment, R¹ and R^{1'} can be -NCOCH₃, -COOH or contain a leaving group such as halogen, ester, acid chloride, mesylate, tosylate, triflate, among others.

In a preferred embodiment the R² linker can contain a leaving group in the terminal position, which is a halogen, ester, acid chloride, mesylate, tosylate, triflate, among others.

The compound of general formula (V) reacts with the spacer R² in the presence of a solvent, at a temperature between 30 and 110 ºC.

In a preferred embodiment, *N,N*-dimethylacetamide, tetrahydrofuran, water, methanol or a mixture of several solvents is used as a solvent. In a more preferred embodiment, the solvents used are ethyl acetate, *N,N*-dimethylacetamide or water.

In a more preferred embodiment, the temperature is set between 40 and 70 ºC.

In a more preferred embodiment, the reaction is performed in the presence of a base such as triethylamine.

An alternative for the preparation of the compounds of general formula (II) consists in a reaction between a compound of general formula (VI) with another compound of general formula (V), where the compound (VI) corresponds to:

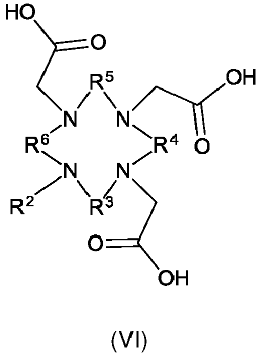

R², R³, R⁴, R⁵ and R⁶ are defined as above.

In a more preferred embodiment, R² may contain a leaving group in the terminal position which is a halogen, ester, acid chloride, mesylate, tosylate, triflate, among others.

The compound of general formula (VI) reacts with another compound of general formula (V) previously defined, in the presence of a solvent, at a temperature between 30 and 110 ºC, preferably between 40 and 70 ºC.

In a preferred embodiment, when solvent is used, this is *N,N*-dimethylacetamide, tetrahydrofuran, ethyl acetate, water, methanol or a mixture of several solvents. In a more preferred embodiment, the mixture used is *N,N*-dimethylacetamide/water.

In a more preferred embodiment, the reaction is carried out using triethylamine as a base.

The compound of general formula (VI) can be obtained by reacting a compound of general formula (IV) described above with a linker R² which is defined as R², in the presence of a solvent, by heating between 30 and 110 ºC, preferably between 40 and 70 ºC.

In a preferred embodiment the R² linker may contain a leaving group which is a halogen, ester, acid chloride, mesilate, tosilate, triflate, among others.

In a preferred embodiment, when solvent is used, this is *N,N*-dimethylacetamide, tetrahydrofuran, water, methanol or a mixture of several solvents. In a more preferred embodiment, the solvents used are water, ethyl acetate or methanol.

In a more preferred embodiment, the reaction is performed using triethylamine as a base.

In all the described procedures, the compounds can be isolated or not.

In all the described procedures, the compounds can be purified by methods commonly known to a person skilled in the art.

Throughout the description and claims the word "comprises" and its variants are not intended to exclude other technical characteristics, additives, components or steps. For those skilled in the art, other objects, advantages and characteristics of the invention will emerge partly from the description and partly from the practice of the invention. The following examples and figures are provided for illustration purposes and are not intended to be limitations of the present invention.

### BRIEF DESCRIPTION OF THE FIGURES

FIG. 1. MRI calibrator phantom study.
FIG. 2. Setting of the ratio of intensity to concentration of the contrast medium containing the compound (8).
FIG. 3. Images of the contrast medium containing the compound (8), over time.
FIG. 4. Distribution and elimination curves of the contrast medium containing the compound (8), in the kidneys and bladder.
FIG. 5. Visualisation of the liver in MRI when administering the contrast medium containing the compound (8).
FIG.6. Distribution curve of the contrast medium containing the compound (8), in the liver.
FIG. 7. Images obtained with PET/CT on administering the contrast medium containing the compound (7).

### EXAMPLES OF THE INVENTION

### Example 1: General synthetic procedure for the invention compounds 7, 8 and 9 and their intermediates 1-6

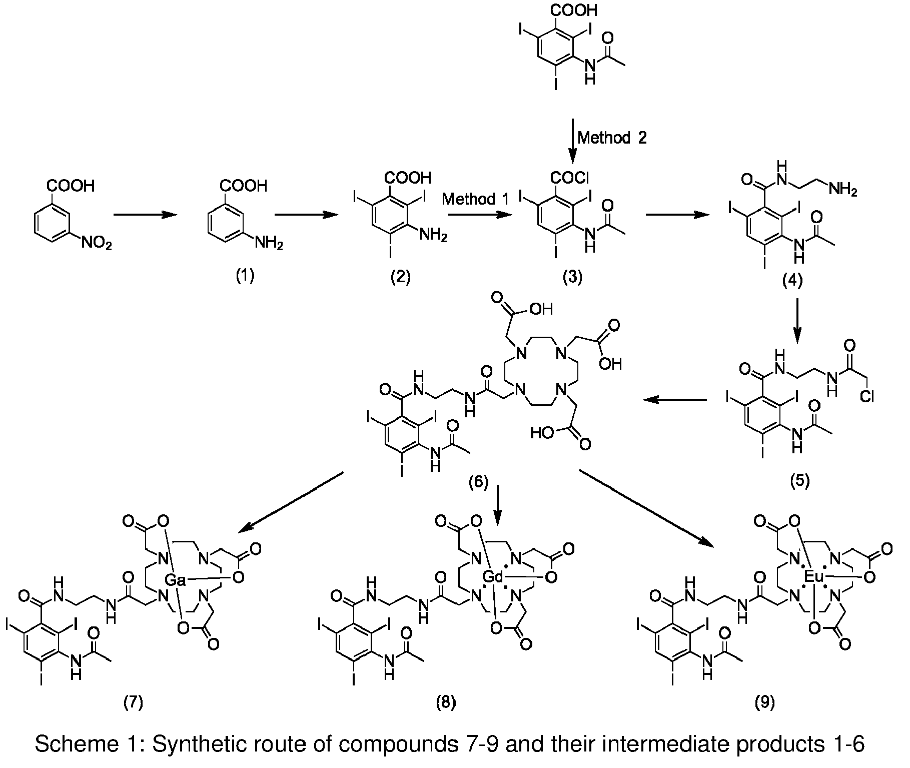

### Compound (1): 3-Aminobenzoic acid

A solution of 3-nitrobenzoic acid (150 g) with sodium carbonate (31.8 g) adjusted to pH 5 with 5% hydrochloric acid was hydrogenated at a pressure of 3-4 bars and a temperature limit of 90°C. Once the reaction had finished, it was precipitated at 65 °C by addition of concentrated sulphuric acid until the pH dropped to 1. Yield: 90%. ESI-MS (m/z): (ES+) 138 [*M*+*H*]⁺*,* 139 [*M*+*2H*]²⁺. (ES-) 136 [*M-H*]⁻. Elemental analysis (C₇H₇NO₂): Theoretical %C 61.31 %H 5.15 %N 10.21; Found %C 61.05 %H 5.18 %N 10.18.

### Compound (2): 3-Amino-2,4,6-triyodobenzoic acid

The compound **(1)** (53.2 g) was dissolved in 250 mL of water by adjusting the pH to 7.0 with sodium carbonate. A two streams addition was made over a diluted solution of hydrochloric acid for one hour; one of these was the solution of the compound previously prepared and the other was an iodinated solution (iodine monochloride stabilized with hydrochloric acid). Once the reaction was completed, a 38 to 40 % of sodium bisulphite aqueous solution was added, filtered and washed with three portions of 30 mL of water.

The purification of the product was carried out by dissolving the product in 300 mL of a saturated solution of sodium bicarbonate in water and treating it with 0.5 g of powdered carbon at 50 ºC. The compound was then precipitated by addition of concentrated hydrochloric acid at a pH below 1 and then it was cooled, filtered and dried under vacuum. Yield: 60%. ESI-MS (m/z): (ES+) 516 [*M*+*H*]⁺, 517 [*M*+*2H*]²⁺*,* 538 [*M*+*Na*]⁺*.* Elemental analysis (C₇H₄I₃NO₂): Theoretical %C 16.33 %H 0.78 %N 2.72; Found %C 16.11 %H 0.71 %N 2.80.

### Compound (3): 3-Acetylamino-2,4,6-triiodobenzoyl chloride

Method 1: The compound **(2)** was dissolved (5 g) in 25 mL of ethyl acetate at room temperature. Then, 1.5 mL of thionyl chloride were added for 30 minutes. Then the excess of thionyl chloride and ethyl acetate was removed until dryness, obtaining a brown solid. Next, 4 g of the acid chloride formed were dissolved in 3.2 mL of *N,N-*dimethylacetamide at room temperature and a solution of acetyl chloride (1.13 mL) was added onto 2.5 mL of *N,N*-dimethylacetamide during 30 minutes. The mixture was stirred at room temperature for one hour. The isolation of the product was carried out by precipitation, for which the previous mixture was added over 90 mL of water and the obtained solid was filtered and washed with water (3 x 40 mL). Yield: 56%

Method 2: Acetrizoic acid (20 g) was dissolved in 200 mL of ethyl acetate at room temperature. This solution was refluxed and thionyl chloride (7 mL) was added during half an hour. When the reaction is complete, 150 mL of the solution were distilled and 100 mL of ethyl acetate were added, and the solvent was removed until dryness. Yield: 94 %. Elemental analysis (C₉H₅ClI₃NO₂): Theoretical %C 18.79 %H 0.88 %N 2.43; Found %C 18.82 %H 0.95 %N 2.50.

### Compound (4): 3-(Acetylamino)-N-(2-aminoethyl)-2,4,6-triiodobenzamide

The obtained solid **(3)** in the previous step was dissolved in 150 mL of *N,N-*dimethylacetamide and added over ethylenediamine (34 mL) during 4-5 hours. Once the addition was finished, it was poured over 280 mL of water, obtaining a white solid, which was filtered and dried under vacuum. Yield: 77%. ESI-MS (m/z): (ES+) 600 [*M*+*H*]⁺, 601 [*M*+*2H*]²⁺*.* Elemental analysis (C₁₁H₁₂I₃N₃O₂): Theoretical %C 22.06 %H 2.02 %N 7.02; Found %C 22.14 %H 2.11 %N 6.96.

### Compound (5): 1-[2-(3-Acetylamino-2,4,6-triiodobenzoylamino)aminoethyl]-2-chloro-1-ethanone

The product **(4)** was suspended (13.31 g) in 130 mL of *N,N*-dimethylacetamide at room temperature. Then, triethylamine (2.72 mL) was added and heated to 70 ºC. Then, chloroacetyl chloride (2.66 mL) was added during 30 minutes and once the reaction is complete, the product is poured over 150 mL of water, obtaining a white solid that is filtered and dried under vacuum. Yield: 78%. ESI-MS (m/z): (ES+) 676 [*M*]⁺, 677 [*M*+*H*]⁺, 678 [*M*+*2H*]²⁺*.* (ES-) 674 [*M-2H*]²⁻*,* 675 [*M-H*]⁻. Elemental analysis (C₁₃H₁₃ClI₃N₃O₃): Theoretical %C 23.12 %H 1.94 %N 6.22; Found %C 23.04 %H 2.05 %N 6.31.

### Compound (6): {10-{2-[2-(3-Acetylamino-2,4,6-triiodobenzylamino) aminoethyl]-2-oxoethyl}-1,4,7,10-tetraaza-1-cyclodecyl}-1,4,7 triacetic acid

Product **(5)** (11.69 g) was dissolved in 110 mL of *N,N*-dimethylacetamide at room temperature. Triethylamine (1.83 mL) was added and heated to 50 ºC. After that, the trisodium salt of 1,4,7,10-tetraazacyclodecane-1,4,7-triaacetic acid (DO3A-3Na) (10.02 g) was added slowly. To increase the solubility of the DO3A-3Na in the mixture, water was added until it was completely dissolved, maintaining the temperature of the reaction. Purification was carried out by nanofiltration at a constant volume. The product obtained was then precipitated with 200 mL acetone, obtaining a white solid that was filtered and dried under vacuum. Yield: 84%. ESI-MS (m/z): (ES+) 986 [*M*+*H*]⁺, 987 [*M*+*2H*]²⁺, 988 [*M*+*3H*]³⁺*.* Elemental analysis (C₂₇H₃₈I₃N₇O₉): Theoretical %C 30.89 %H 4.57 %N 9.01; Found %C 30.99 %H 4.23 %N 8.70.

### Compound (7): Gallium {10-{2-[2-(3-acetylamino-2,4,6-triiodophenylamino) aminoethyl]-2-oxoethyl}-1,4,7,10-tetraaza-1-cyclodecyl}-1,4,7-triacetate

Product **(6)** (12.43 g) was dissolved in 125 mL of water and heated to 80 ºC. After that, gallium chloride (2.67 g) was added maintaining the pH of the reaction between 2 and 5 with hydrochloric acid. When the reaction was finished, the product was purified by treatment with resins (IRA-67 and IR-120). Later, it was precipitated in a mixture of ethanol/acetone obtaining a white precipitate that was filtered and dried under vacuum. Yield: 80%. m.p.: 287.9 ºC. IR (ATR): 1651, 1558, 1539, 1520, 1310, 1082, 922, 729 cm⁻¹. ¹H NMR (500 MHz, D₂O) δ: 8.52 (s, 1H, H-Ph); 3.98-3.46 (m, 12H, 6CH₂); 3.45 (s, 6H, 3CH₂); 3.44-3.06 (m, 10H, 5CH₂); 2.21 (s, 3H, CH₃). ¹³CNMR (125 MHz, D₂O) δ: 175.0 (CO); 174.5 (CO); 173.6 (CO); 173.5 (CO); 172.5 (CO); 168.6 (CO); 148.8 (CH-Ph); 147.9 (C-Ph); 141.6 (C-Ph); 100.1 (C-Ph); 96.9 (C-Ph); 91.4 (C-Ph); 63.3 (CH₂); 61.8 (CH₂); 59.4 (3CH₂); 57.3 (CH₂); 57.2 (CH₂); 55.9 (2CH₂); 54.6 (2CH₂); 53.9 (2CH₂); 44.8 (CH₂); 22.1 (CH₃). ESI-MS (m/z): (ES+) 1054 [*M*+*2H*]²⁺, 1055 [*M*+*3H*]³⁺*,* 1056 [*M*+*4H*]⁴⁺*.* (ES-) 1050 [*M-2H*]²⁻*,* 1051 [*M-H*]⁻, 1052 [*M*]⁻. Elemental analysis (C₂₇H₃₅Gal₃N₇O₉): Theoretical %C 30.37 %H 3.73 %N 8.85; Found %C 30.32 %H 4.31 %N 9.18.

### Compound (8): Gadolinium {10-{2-[2-(3-acetylamino-2,4,6-triiodophenylamino) aminoethyl]-2-oxoethyl}-1,4,7,10-tetraaza-1-cyclodecyl}-1,4,7-triacetate

The compound **(6)** (4.5 g) was dissolved in 45 mL of water and the resulting solution was heated to 80 ºC. Then gadolinium oxide (0.84 g) was added, maintaining the pH of the reaction between 4.0 and 4.5 by addition of concentrated hydrochloric acid. Once the reaction was finished, the product was purified by treatment with resins (IRA-67 and IR-120). Subsequently, it was precipitated in an ethanol/acetone mixture, obtaining a white solid that was filtered and dried under vacuum. Yield: 91%. m.p.: 290.9 ºC. IR (ATR): 1601, 1397, 1317, 1281, 1242, 1086, 714 cm⁻¹. ESI-MS (m/z): (ES+) 1140 [*M*]⁺, 1141 [*M*+*H*]⁺*.* (EN-) 1139 [*M*-*1H*]⁻. Elemental analysis (C₂₇H₃₅GdI₃N70₉): Theoretical %C 26.37 %H 3.69 %N 7.97; Found %C 26.11 %H 3.60 %N 7.85.

### Compound (9): Europium {10-{2-[2-(3-acetylamino-2,4,6-triiodophenylamino) aminoethyl]-2-oxoethyl}-1,4,7,10-tetraaza-1-cyclodecyl}-1,4,7-trioacetate

The compound **(6)** (1.5 g) was dissolved in 20 mL of water. The solution was heated to 80 ºC and europium oxide (0.27 g) was added. The pH of the reaction was maintained between 4.0 and 4.5 with concentrated hydrochloric acid. Once the reaction was finished, the product was purified by treatment with resins (IRA-67 and IR-120). Subsequently it was precipitated in an ethanol/acetone mixture. A white precipitate was obtained, which was filtered and dried under vacuum. Yield: 87%. m.p.: 294.0 ºC. IR (ATR): 1595, 1368, 1315, 1279, 1238, 1084, 935, 712 cm⁻¹. ¹H NMR (300 MHz, DMSO-*d₆*) δ: 9.90 (bs, 1H, NH); 8.58 (bs, 1H, H-Ph); 8.25 (m, 2H, 2NH); 3.25-2.75 (m, 28H, 14CH₂); 2.03 (s, 3H, CH₃). ¹³C NMR (75 MHz, DMSO-*d₆*) δ: 175.0 (CO); 174.5 (CO); 173.6 (CO); 173.5 (CO); 172.5 (CO); 168.6 (CO); 148.8 (CH-Ph); 147.9 (C-Ph); 141.6 (C-Ph); 100.1 (C-Ph); 96.9 (C-Ph); 91.4 (C-Ph); 63.3 (CH₂); 61.8 (CH₂); 59.4 (3CH₂); 57.3 (CH₂); 57.2 (CH₂); 55.9 (2CH₂); 54.6 (2CH₂); 53.9 (2CH₂); 44.8 (CH₂); 22.1 (CH₃). ESI-MS (m/z): (ES+) 1136 [*M*+*H*]⁺, 1158 [*M*+*Na*]⁺*.* (ES-) 1134 [*M-H*]⁻. Elemental analysis (C₂₇H₃₅EuI₃N₇O₉): Theoretical 27.35 %C 3.77 %H 7.98 %N; Found 27.58 %C 4.32 %H 8.22 %N.

### Example 2: General procedure of synthesis of the compounds of the invention 14, 15 and 16 and their intermediates 10-13.

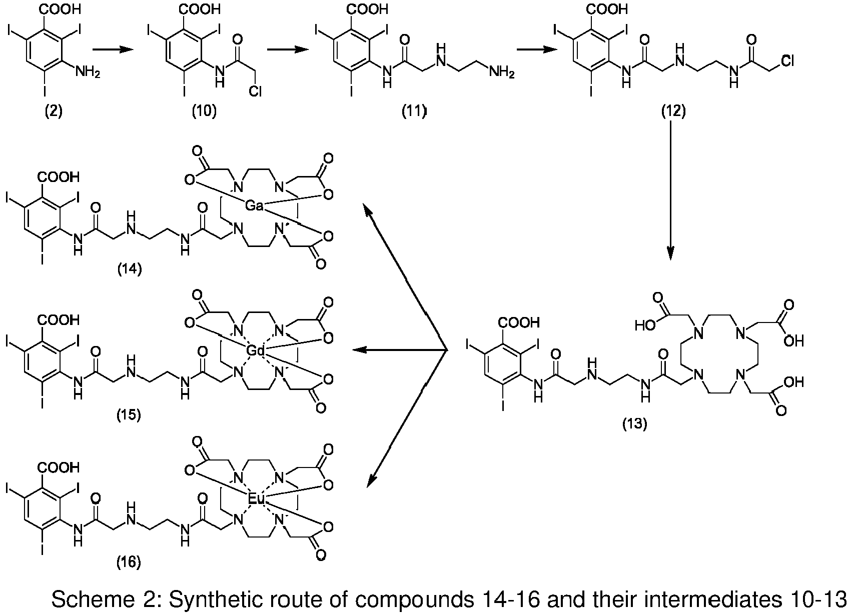

### Compound (10): 3-(2-Chloroacetylamino)-2,4,6-triiodobenzoic acid

Compound **(2)** (5 g) was dissolved in 50 mL of *N,N*-dimethylacetamide at room temperature. This solution was heated to 70 ºC and the chloroacetyl chloride (1 mL) was added for 30 minutes. When the reaction was finished, the solvent was evaporated, and the product was dissolved in water. Then, the pH was reduced below 1 with concentrated hydrochloric acid precipitating a white solid that was filtered and dried under vacuum. Yield: 93%. ESI-MS (m/z): (ES+) 592 [*M+H*]⁺, 593 [*M+2H*]²⁺*,* 615 [*M+H+Na*]⁺*.* Elemental analysis (C₉H₅ClI₃NO₃): Theoretical %C 18.28 %H 0.85 %N 2.37; Found %C 18.31 %H 0.92 %N 2.30.

### Compound (11): 3-[2-(2-Aminoethylamino)acetylamino]-2,4,6-triiodobenzoic acid

Product **(10)** (3 g) was dissolved in 30 mL of basic aqueous medium and added over ethylenediamine (8.5 mL) for 4 hours. Once the reaction was finished, the pH was reduced below 1 with concentrated hydrochloric acid precipitating a white solid that was filtered and dried under vacuum. Yield: 83%. ESI-MS (m/z): (ES+) 616 [*M+H*]⁺, 617 [*M+2H*]²⁺, 638 [*M+Na*]⁺*.* Elemental analysis (C₁₁H₁₂I₃N₃O₃): Theoretical %C 21.48 %H 1.97 %N 6.83; Found %C 21.40 %H 1.88 %N 6.80.

### Compound (12): 3-{2-[2-(2-Chloroacetylamino)aminoethyl]acetylamino}-2,4,6-triiodobenzoic acid

Compound **(11)** (5 g) was dissolved in 50 mL of *N,N*-dimethylacetamide at room temperature. The solution was then heated to 70°C and chloroacetyl chloride (1.40 mL) was added for 30 minutes. Once the reaction was finished, the solvent was removed, and the product was dissolved in 50 mL of water. Afterwards, the pH was reduced below 1 with concentrated hydrochloric acid obtaining a white solid that was filtered and dried under vacuum. Yield: 89%. ESI-MS (m/z): (ES+) 692 [*M+H*]⁺, 693 [*M+2H*]²⁺*,* 714 [*M+Na*]⁺*.* (ES-) 690 [*M-H*]⁻. Elemental analysis (C₁₃H₁₃ClI₃N₃O₄): Theoretical %C 22.58 %H 1.90 %N 6.08; Found %C 22.65 %H 1.89 %N 6.13.

### Compound (13): 3-{2-{2-{2-[4,7,10-Tris(carboxymethyl)-1,4,7,10-tetraaza-1-cyclododecyl]acetylamino}aminoethyl}acetylamino}2,4,6-triiodobenzoic acid

Compound **(12)** (4 g) was dissolved in 40 mL of basic aqueous medium at room temperature. The solution was heated to 60 ºC and DO3A-3Na (2.6 g) was added. Once the reaction was complete, the pH was reduced below 1 with concentrated hydrochloric acid, precipitating a white solid. The product was purified by nanofiltration at a constant volume and then precipitated with acetone, obtaining a white solid that was filtered and dried under vacuum. Yield: 67%. ESI-MS (m/z): (ES+) 1001 [*M*]⁺, 1002 [*M*+*H*]⁺, 1003 [*M*+*2H*]²⁺*.* (ES-) 1000 [*M-H*]⁻. Elemental analysis (C₂₇H₃₈I₃N₇O₁₀): Theoretical %C 32.39 %H 3.83 %N 9.79; Found %C 32.58 %H 3.95 %N 9.83.

### General procedure for compounds 14, 15 and 16

Product **(13)** was dissolved in water and heated to 80 ºC. The corresponding metal derivative was added, maintaining the pH of the reaction with hydrochloric acid. Once the reaction was finished, the product was purified by treatment with resins (IRA-67 and IR-120). Later it was precipitated in a mixture of ethanol/acetone obtaining a white solid that was filtered and dried under vacuum.

### Compound (14): Gallium {10-{2-{2-[2-(3-Carboxy-2,4,6-triiodophenylamino)-2-oxoethylamino] aminoethyl}-2-oxoethyl}-1,4,7,10-tetraaza-1-cyclodododecyl}-1,4,7-triacetate

Reagents: compound **(13)** (4 g), gallium chloride (0.71 g) and water (40 mL). Reaction conditions: pH of the reaction 2.0 - 5.0. Yield: 89%. m.p.: 286.8 ºC. IR (ATR): 3340, 3215, 3160, 1687, 1563, 1515, 1509, 1340, 1080, 930, 714 cm⁻¹. ¹H RMN: (400 MHz, DMSO-*d₆*) δ: 8.38 (s, 1H, COOH); 8.20 (bs, 4H, 3NH, H-Ph); 3.61 - 3.54 (m, 8H, 4CH₂); 3.48 - 3.44 (m, 2H, CH₂), 3.23 (t, *J* = 7.2 Hz, 2H, CH₂); 2.84 (t, *J* = 7.2 Hz, 2H, CH₂); 2.80 - 2.75 (m, 16H, 8 CH₂). ¹³C RMN: (100 MHz, DMSO-d₆) δ: 175.1 (CO); 175.0 (CO); 174.8 (CO); 174.5 (CO); 172.1 (CO); 171.9 (CO); 155.5 (C-Ph); 145.3 (CH-Ph); 145.2 (C-Ph); 99.3 (C-Ph); 99.1 (C-Ph); 98.6 (C-Ph); 57.8 (CH₂); 54.1 (6 CH₂); 52.3 (CH₂); 51.9 (2 CH₂); 51.5 (CH₂); 50.8 (CH₂); 50.1 (CH₂); 49.8 (CH₂); 41.2 (CH₂). ESI-MS (m/z): (ES+) 1068 [*M*]⁺, 1069 [*M+H*]⁺*,* 1070 [*M+2H*]²⁺*.* (ES-) 1067 [*M-H*]⁻. Basic analysis (C₂₇H₃₅GaI₃N₇O₁₀): Theoretical %C 30.36 %H 3.30 %N 9.18; Found %C 30.41 %H 3.38 %N 9.02.

### Compound (15): Gadolinium {10-{2-{2-[2-(3-Carboxy-2,4,6-triiodophenylamino)-2-oxoethylamino] aminoethyl}-2-oxoethyl}-1,4,7,10-tetraaza-1-cyclodododecyl}-1,4,7-triacetate

Reagents: compound **(13)** (4 g) and gadolinium oxide (0.72 g). Reaction conditions: pH of the reaction 4.0 - 4.5 Yield: 84%. m.p.: 288.7 ºC. IR (ATR): 3342, 3219, 3151, 1616, 1404, 1321, 1297, 1212, 1112, 708 cm⁻¹. ESI-MS (m/z): (ES+) 1055 [*M*]⁺, 1056 [*M+H*]⁺, 1057 [*M+2H*]²⁺*.* (ES-) 1054 [*M-H*]⁻. Elemental analysis (C₂₇H₃₅GdI₃N₇O₁₀): Theoretical %C 28.06 %H 3.05 %N 8.48; Found %C 28.19 %H 2.95 %N 8.59

### Compound (16): Europium {10-{2-{2-[2-(3-Carboxy-2,4,6-triiodophenylamino)-2-oxoethylamino] aminoethyl}-2-oxoethyl}-1,4,7,10-tetraaza-1-cyclodododecyl}-1,4,7-triacetate

Reagents: compound **(13)** (4 g) and europium oxide (0.70 g). Reaction conditions: pH of the reaction 4.0 - 4.5. Yield: 87%. m.p.: 293.4 ºC. IR (ATR): 3353, 3232, 3155, 1603, 1379, 1322, 1292, 1223, 1086, 932, 724 cm⁻¹. ¹H RMN: (400 MHz, DMSO-*d₆*) δ: 8.29 (s, 1H, COOH); 8.15 (bs, 4H, 3NH, H-Ph); 3.75 - 3.41 (m, 8H (4 CH₂); 3.31 - 3.20 (m, 2H, CH₂), 3.15 (t, *J* = 6.9 Hz, 2H, CH₂); 2.69 (t, *J* = 6.9 Hz, 2H, CH₂); 2.91 - 2.69 (m, 16H, 8 CH₂). ¹³C RMN: (100 MHz, DMSO-d₆) δ: 175.8 (CO); 175.4 (CO); 174.8 (CO); 174.5 (CO); 172.9 (CO); 170.1 (CO); 160.5 (C-Ph); 143.2 (CH-Ph); 140.9 (C-Ph); 97.8 (C-Ph); 97.1 (C-Ph); 96.4 (C-Ph); 55.4 (CH₂); 52.8 (6 CH₂); 52.4 (CH₂); 51.5 (2CH₂); 50.9 (CH₂); 50.5 (CH₂); 49.6 (CH₂); 48.2 (CH₂); 40.6 (CH₂). ESI-MS (m/z): (ES+) 1151 [*M+H*]⁺*,* 1152 [*M+2H*]²⁺*.* (ES-) 1149 [*M-H*]. Elemental analysis (C₂₇H₃₅EuI₃N₇O₁₀): Theoretical %C 28.19 %H 3.07 %N 8.52; Found %C 28.07 %H 3.20 %N 8.61.

### Example 3: Study of albumin affinity, osmolality and partition coefficient (P)

The albumin affinity test was carried out by *in-vitro* studies using Thermo Scientific^{™} "Single-Use RED Plate with 8K MWCO Inserts" 96-well plate, Bovine Serum Albumin (BSA) and HEPES. The technique of the devices is based on the process of equilibrium dialysis. The tests were carried out in a HEPES buffer with a pH of 7.4. In a typical test, following the manufacturer's measurement procedure, 400 µL of a solution containing the compound to be studied and BSA in concentrations of 0.25 mM and 0.6 mM respectively, in HEPES buffer, are added to the sample well. Additionally, 600 µL of HEPES buffer is added in the buffer well. After shaking the system for five hours at 300 rpm at a constant temperature of 37 ºC, the concentration of the compound in each well is analysed. This analysis was performed using an Agilent ICP-MS (7900 Series) and with these concentration data the percentage of affinity of the studied compounds was determined. The procedure was also performed for two reference compounds such as gadobutrol and acetrizoic acid, because gadobutrol has a very low affinity for albumin, whereas acetrizoic acid has a higher affinity for albumin.

The osmolality test was performed using freezing point equipment using solutions of the compounds at a concentration of 0.1 M. The procedure was also carried out for two reference compounds such as gadobutrol and acetrizoic acid; due to gadobutrol has a low osmolality compared to acetrizoic acid which has a high osmolality. The test for determining the partition coefficient was carried out on solutions of the compounds studied at a concentration of 5 mM dissolved in TRIS buffer adjusted to pH 7.6. The study was performed in 1-butanol/water and after shaking for 30 minutes at 230 rpm, the concentration of the compounds in each phase was analysed using an Agilent ICP-MS (Series 7900). With these data the partition coefficient of the studied compounds was determined. The procedure was also carried out for two reference compounds, gadobutrol and acetrizoic acid, gadobutrol has a more hydrophilic character in accordance with the contrast media used in MRI on the contrary to acetrizoic acid which has a less hydrophilic character as the compounds used in X-ray explorations.

**Table 1: Properties of the studied contrast media**

| **Compound** | **Albumin affinity (%)** | **Osmolality (mOs/Kg)** | **Partition Coefficient (Log P)** |
|---|---|---|---|
| Gadobutrol | Not detectable | 93.33 | -2.309 |
| (7) | 8.89 | 123.43 | -1.426 |
| (8) | 11.16 | 117.58 | -0.971 |
| (9) | 10.45 | 125.12 | -1.032 |
| (14) | 18.53 | 150.42 | -0.675 |
| (15) | 20.54 | 155.38 | -0.581 |
| (16) | 19.12 | 148.12 | -0.597 |
| Acetrizoic acid | 39.16 | 258.47 | -0.482 |

### Example 4: Measurement of IC₅₀ in vitro

IC₅₀ values were obtained by measuring cell viability in V79 fibroblast cultures as an alternative to animal experimentation. The values determined are summarized in the following table:

**Table 2: IC₅₀ determination**

| **Compound** | **IC₅₀ (mg/mL)** |
|---|---|
| Gadobutrol | >75 |
| (7) | 8.64 |
| (8) | >75 |
| (9) | >75 |
| (14) | 10.12 |
| (15) | >75 |
| (16) | >75 |
| Acetrizoic acid | 4.04 |

### Example 5: Calibrator (phantom) test in MRI

The calibrator (phantom) test with solutions of the compound (**8**) was performed in a 7T animal MRI equipment. The relationship between the concentration of the compound (0, 1, 3, 10, 25 and 50 mM) and the signal intensity was studied.

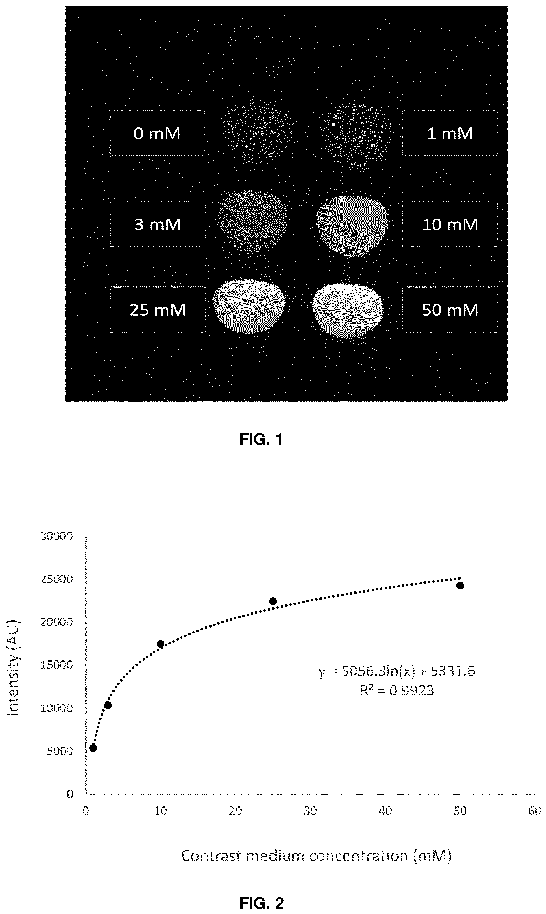

The image in figure 1 was obtained by studying the T₁ parameter for a TR value of 200 ms and a rotation angle of 90º. Analysing the relationship of the intensity *versus* the concentration of the contrast medium, a logarithmic adjustment was obtained, which is shown in figure 2.

### Example 6: Image obtained in MRI

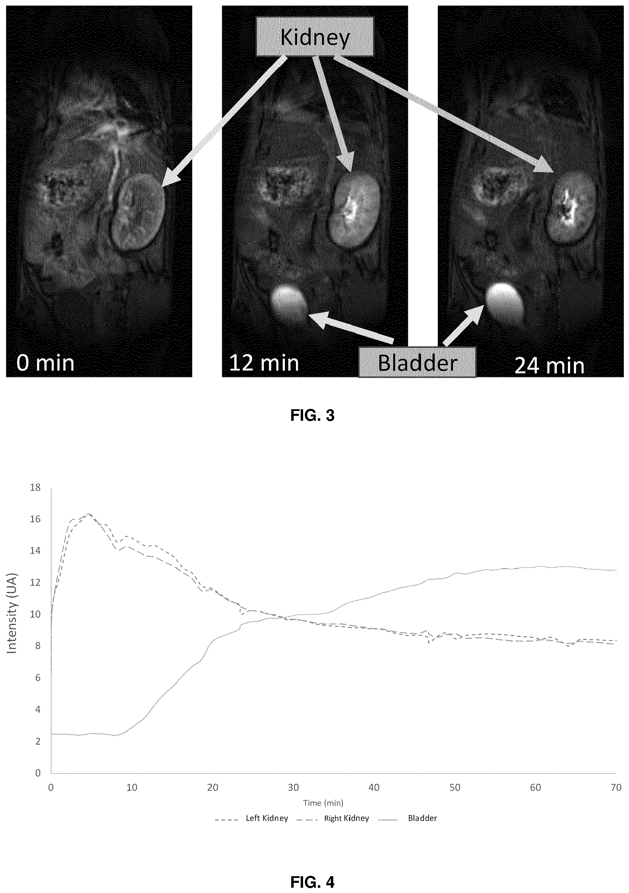

The test was carried out in a 7T animal magnetic resonance equipment by administering the contrast medium containing the compound (**8**) at a dose of 0.1 mmol/Kg to mice. In figures 3 and 4 it can be seen that the contrast media has an elimination via the kidneys, with an exponential distribution and elimination over time in the kidneys and bladder.

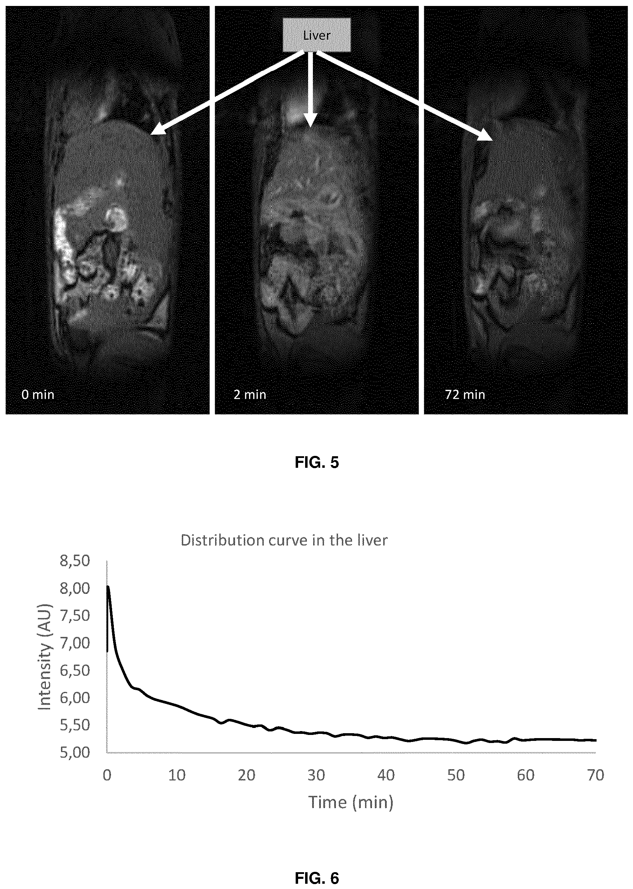

An additional study has shown that by administering the contrast medium containing the compound (**8**) and observing the signal in MRI, visualisation of the liver is possible. As can be seen in figures 5 and 6, the distribution in the liver is also exponential.

### Example 7: Image obtained in PET/CT

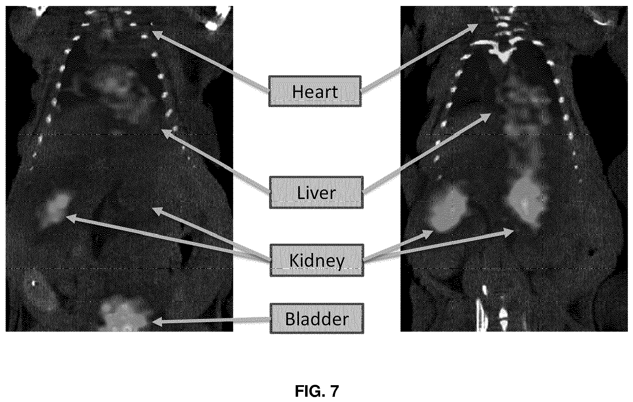

After administering a 130 µCi solution of the contrast medium containing the gallium 68-labelled compound (**7**) to a mice in an animal PET/CT scanner, images of different cuts-sections are obtained in which the heart, liver, kidneys and bladder are differentiated (Figure 7).

## Claims

1. Compound of formula (I): 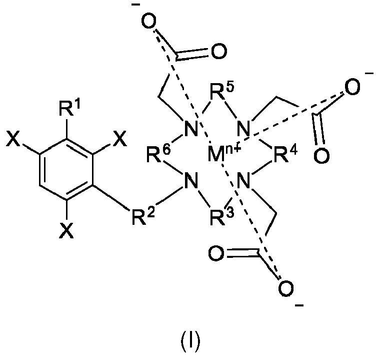 where
R¹ is selected from alkyl C₁-C₁₂, alkenyl C₂-C₁₂, aryl C₅-C₁₀, heteroaryl C₅-C₁₀, halogen, -OR⁷, -NR⁷R⁸, -CN, -C(O)R⁷, -C(O)OR⁷, -OC(O)R⁷, -C(O)NR⁷R⁸, -NHC(O)R⁷, -SO₂R⁷, - SO₂NR⁷R⁸ or -NO₂, where R⁷ and R⁸ are independently selected from H, alkyl C₁-C₁₂, alkyl C₂-C₁₂, aryl C₅-C₁₀ or heteroaryl C₅-C₁₀;
X is a halogen, preferably bromine or iodine;
R² is the following group -(CH₂)ₘW(CH₂)ₚY(CH₂)_{q}Z(CH₂)ᵣ- , where m, p, q and r are integers independently selected from 0 to 10, and W, Y, Z are independently selected from the following groups: -O-, -NR⁹-, -C(O)-, -C(O)O-, -C(O)NR¹⁰-, -NR¹¹C(O)-, -CH₂-, - C=, -SO₂-, -SO₂N(R¹²)-, aryl C₅-C₁₀ or heteroaryl C₅-C₁₀, where R⁹, R¹⁰, R¹¹ and R¹² are independently selected from H or alkyl C₁-C₆;
R³, R⁴, R⁵ and R⁶ are independently selected from alkyl C₁-C₁₂, alkenyl C₂-C₁₂, aryl C₅-C₁₀ or heteroaryl C₅-C₁₀;
M is a metal selected from gadolinium, gallium, europium, ytterbium, dysprosium, manganese, strontium, thallium, lutetium, tungsten, germanium, copper, yttrium, indium, scandium or any of their possible isotopes, and n can be 2 or 3;
and its pharmaceutically acceptable salts, isomers and solvates.

2. Compound according to claim 1, where R¹ is selected from -NHC(O)R⁷ or - C(O)OR⁷, with R⁷ being hydrogen or C₁-C₆ alkyl.

3. Compound according to either claim 1 or 2, where X is iodine.

4. Compound according to any of the claims 1 to 3, where m is 0.

5. Compound according to any of claims 1 to 4, where W is selected from - C(O)NR¹⁰- or -NR¹¹C(O)-, with R¹⁰ and R¹¹ preferably H.

6. Compound according to any of the claims 1 to 5, where Y is selected from -NR⁹- or -NR¹¹C(O)-, with R⁹ and R¹¹ preferably H.

7. Compound according to any of claims 1 to 6, where Z is selected from -CH₂- or - NR¹¹C(O)-, with R¹¹ preferably being H.

8. Compound according to any of the claims 1 to 7, where p is selected from 1 to 2, q is selected from 0 to 2 and r is selected from 0 to 1.

9. Compound according to any of the claims 1 to 8, where R³, R⁴, R⁵ and R⁶ are an equal or different C₁-C₆ alkyl, preferably a C2 alkyl.

10. Compound according to claim 1 selected from the following list:
- Gadolinium {10-{2-[2-(3-acetylamino-2,4,6-triiodophenylamino)aminoethyl]-2-oxoethyl}-1,4,7,10-tetraaza-1-cyclododecyl}-1,4,7-triacetate
- ⁶⁷Gallium {10-{2-[2-(3-acetylamino-2,4,6-triiodophenylamino)aminoethyl]-2-oxoethyl}-1,4,7,10-tetraaza-1-cyclododecyl}-1,4,7-triacetate
- ⁶⁸Gallium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Europium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Dysprosium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Ytterbium {10-{2-[2-(3-acetylamino-2,4,6-triiodophenylamino)aminoethyl]-2-oxoethyl}-1,4,7,10-tetraaza-1-cyclododecyl}-1,4,7-triacetate
- Gadolinium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- ⁶⁷Gallium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- ⁶⁷Gallium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Europium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Dysprosium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate
- Ytterbium {10-{2-{2-[2-(3-carboxy-2,4,6-triiodophenylamino)-2-oxoethylamino] aminoethyl}-2-oxoethyl}-1,4,7,10-tetraaza-1-cyclododecyl}-1,4,7-triacetate

11. Pharmaceutical composition comprising a compound of formula (I) according to any of the Claims 1 to 10 with at least one pharmaceutically acceptable excipient, adjuvant and/or vehicle.

12. Compound of formula (I) according to any of the claims 1 to 10 for its use as a drug.

13. Compound of formula (I) according to any of the claims 1 to 10 for the use in the treatment and/or diagnosis of tumours.

14. Contrast medium for imaging organs and/or tissues of the human or animal body using nuclear magnetic resonance, X-ray, PET or SPECT, or combinations of these techniques, comprising at least one compound of formula (I) according to any of the claims 1 to 10.

15. Procedure for obtaining a compound of formula (I) according to any of the claims 1 to 10 which comprise making a compound of formula (II): 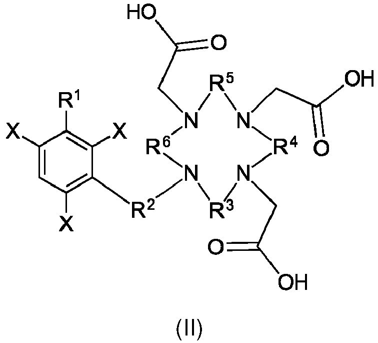
where R¹, R², R³, R⁴, R⁵ and R⁶ are defined as in claim 1,
to react with a metal selected from gadolinium, gallium, europium, ytterbium, dysprosium, manganese, strontium, thallium, lutetium, tungsten, germanium, copper, yttrium, indium or scandium or any of their possible isotopes, in the form of an oxide or salt, preferably chloride or bromide, in the presence of a solvent, preferably water or methanol, at a temperature of between 60 and 110 ºC, preferably between 80 and 90 ºC.
